# NEW EUROPEAN PATENT SPECIFICATION

(11) **EP 0 771 523 B2**
(45) Date of publication and mention of the opposition decision: **07.06.2006**
(45) Mention of the grant of the patent: 07.01.1999
(21) Application number: 95202975.9
(22) Date of filing: 02.11.1995
(51) Int. Cl.: A01H 1/02, A01H 5/10

(54) **A cytoplasmic male sterile vegetable plant cell of the compositae family and also a method for obtaining such a plant**
Zytoplasmische männliche-sterile Pflanzenzelle der Compositae Familie und Verfahren zur Herstellung der Pflanze
Cellule d'une plante avec stérilité mâle cytoplasmatique de la famille des Compositae et méthode pour obtenir la plante

(43) Date of publication of application: 07.05.1997
(73) Proprietor: Enza Zaden Beheer B.V., 1600 AA Enkhuizen (NL)
(72) Inventor: Faber, Nanne M., NL-1628 SJ Hoorn (NL); Lambalk, Joep J.M., NL-1462 MG Middenbeemster (NL); Nootebos, Margret G., NL-1602 JD Enkhuizen (NL); Zaal, Monique A.C.M., NL-1689 PJ Zwaag (NL)
(74) Representative: Hooiveld, Arjen Jan Winfried

(56) References cited:
- WO-A-84/03606
- FR-A- 2 628 601
- GB-A- 2 211 205
- US-A- 5 278 057
- FIZIOL RAST, vol. 13, no. 3, 1986, SOFIA, BULGARIA, pages 20-25, XP002000315 NIKOLOV, S KH: "Investigation of the functional activity of chloroplasts isolated from cytoplasmatic male sterile sunflower forms."
- DATABASE WPI Derwent Publications Ltd., London, GB; AN 95-271109, XP002000376 & HU-A-68 646 (DATE KUTATO KOEZPONTJA) 28 July 1995
- JOURNAL OF HEREDITY, vol. 85, no. 3, 1994, pages 233-238, XP002000316 RIESEBERG, L H ET AL: "Cytoplamatic male sterility in sunflower: origin, inheritance and frequency in natural populations"
- Rambaud C. et al., Male-sterile chicory cybrids obtained by intergeneric protoplast fusion, Theor Appl Genet (1993) 87: 347-352
- Rambaud C. et al., Fusion de protoplastes chicorée/tournesol, C.R. Acad. Agric. Fr. (1994) 80, no 7, pp 63-67
- Rambaud C. et al., La stérilité mâle cytoplasmique chez la chicorée industrielle: obtention par fusion de protoplastes et caractérisation moléeculaire. Quel avenir pour l'amélioration des plantes. Paris (1994) pp 483-489
- Kohler et al., Mol. Gen. Genet. (1991) 227: 369-376
- Rambaud et al., Plant Breeding (1997) 116, 481-486
- Galun & Aviv, Methods in Enzymology (1986) 118: 595-611
- Rambaud C. et al., The induction of tetraploidy in chicory (Cichorium intybus L var Magdeburg) by protplast fusion, Euphytica (1992) 62, 63-67
- Rambaud C. et al., Some factors related to protoplast culture and plant regeneration from leaf mesophyll protoplasts of Magdeburg chicory (Cichorium intybus L. var. Magdeburg), Agronomie (1990) 10, 767-772

## Description

The invention relates to a cytoplasmic male sterile vegetable plant of the Compositae family in accordance with the main claim, that is, to the development of new parent lines of such a plant in said family. The invention furthermore relates to a method for obtaining the aforesaid cytoplasmic male sterile plant.

In the plant breeders' world it is customary to combine hereditary properties of two parent lines in the hybrid through crossing. If the two parent lines are self-pollinating, self-pollination is prevented, for example by removing anthers (the so-called emasculating), in order to ensure that the desired hereditary properties of both parents are present in the hybrid. Such a hybrid is also called an F1 hybrid, and it is of great importance to the plant breeders' world. F1 hybrids are usually characterized by a very high degree of uniformity (colour, taste, size and the like), a high vigour and a high yield of plant parts, such as fruits and the like, that can be commercialized. An additional advantage of an F1 hybrid especially for plant breeding companies is moreover the fact that it is not possible to raise products of the same quality by progeny from the seed obtained therefrom (greater heterogeneity and lower yield), so that the company is assured of an inherent protection of its F1 hybrid, as it were. With vegetable plants of the Compositae family the development of parent lines and the commercial production of pure F1 hybrid seed is very problematic, because these vegetable plants are generally self-pollinating and because the anti-self-pollination measures that have been used so far are not only time-consuming and laborious, but have moreover appeared to be insufficiently accurate. Accordingly this latter fact results in seed lots that are not of a uniform quality.

For example, with cabbage plants (Brassica oleracea) it is now possible to produce 100% pure F1 hybrids by using modem techniques. The fact is that in radish (Raphanus sativus), which like cabbage belongs to the Cruciferae family, a cytoplasm causing male sterility has been found, which cytoplasm is also known as Ogura CMS cytoplasm Mitochondria of the Ogura CMS cytoplasm are introduced in Brassica oleracea plants through fusion of protoplasts, whereby both the nucleus and the chloroplasts (chlorophyll granules) originate from normally fertile Brassica oleracea plants. Thus CMS Brassica oleracea plants are obtained. At the moment no F1 hybrids of important vegetable plants of the Compositae family are available yet, in spite of the great market demand. The aforesaid Ogura CMS cytoplasm cannot be used with vegetable plants of the Compositae family, because of the genetic incompatibility between species of said families.

The object of the invention is to introduce cytoplasmic male sterility in a vegetable plant of the Compositae family, and to that end the cytoplasm of such a vegetable plant is provided with mitochondria comprising DNA which at least partially originates from a different species of the Compositae family and which is carrier of stably expressable cytoplasmic male sterility (CMS), whose cells comprise species - specific chloroplast and nuclear genome which are normal for the vegetable plant Surprisingly research has shown that in spite of the strong genetic incompatibility of plant species within the Compositae family, the introduction of CMS into a vegetable plant of the Compositae family from a plant of a different species of the same family, through fusion of protoplasts, is very well possible. Preferably the vegetable plant is phenotypically cytoplasmic male sterile, that is, the male sterility (generally defined as the inability of a plant to produce fertile pollen grains) has manifested itself in the plant Because of the cytoplasmic heredity of CMS all progeny of this male sterile plant posses this unchanged property. It will be apparent that the above form of heriditary transmission of genotypic CMS is of great significance for the efficient development of parent lines of the vegetable species and for the subsequent production of F1 hybrids. It is noted that the plant does not necessarily have to be phenotypically cytoplasmic male stenle, but that it is also possible for the nucleus to possess so-called "repair genes", which phenotypically undo the male sterility, that is, prevent it from manifesting itself in the plant in spite of the presence of DNA in the cytoplasm, which is a carrier of CMS. In that case it will be necessary to keep crossing until a plant possessing phenotypic CMS is obtained. Because a nucleus and chloroplasts of the normal, fertile vegetable plant is present in unchanged condition (in the case of the nucleus this is usually a diploid situation), the product is a 100% pure product.

Publication in Theor.Appl.Genet (Vol.87, pages 347-352 (1993)) entitled "Male-sterile chicory cybrids obtained by intergeneric protoplast fusion" describes an agricultural plant of the "Cichorium intybus cv Magdebourg" type, the cytoplasm of which is provided with mitochondria having DNA originating from Helianthus annuus (CMS-type petiolaris). However, no stably expressable CMS was obtained, nor 100% pure plants as far as the chloroplast and nuclear genome is concerned.

Two other publications of Ramboud et al. (C.R. Acad. Agric. Fr., vol. 80(7): 63-67 (1994) and "Quel avenir pour l'améloriation des plantes ?", Ed. AUDELF-UREF, John Libeey Eurotext, Paris, pages 483-489 (1994)) relate to the same failed attempt to obtain stable male sterile plants of the Compositae family.

In a preferred embodiment of the invention the other species of the Compositae family has been selected from the group consisting of Helianthus spp, and Circium spp. These species have been found to contain CMS. More in particular Helianthus annuus (L) (sunflower) with CMS of the petiolaris type has been selected. Surprisingly the introduction of CMS from these genotypically cytoplasmic male sterile plants can take place in an efficient manner by means of fusion of protoplasts, in spite of the genetic incompatibility. In this connection it is noted that the transmission of CMS from the aforesaid plants to the vegetable plants belonging to the same family cannot be done by using conventional methods, because of crossing barriers.

According to the invention the other CMS-introducing species (the "donor"), which belongs to the Compositae family, may also be a species that does not contain CMS, but which induces a so-called "alloplasmic" form of CMS in the vegetable plant by means of fusion of protoplasts. According to the invention a species of this type is preferably selected from the group consisting of Chrysanthenum, Senecio, Centaurea, Sonchus Hieracium, Tagetes, Dahlia and Aster, Good results have been achieved in accordance with the invention in particular with Chryrsanthenum, more in particular with Sonchus.

In another embodiment according to the invention the vegetable plant has been selected from the group consisting of Cichorium intybus (L.) var. foliasum (Hegi) (chicory), Cichorium endivia (L.) (endive), Lactuca sativa (L.) (lettuce), Scorzonera hispanica (L.) (black salsify), Cynara scolymus (L) (artichoke) and Taraxacum offianale (L.) (dandelion salad). Extensive experiments have shown that in particular Lactuca sativa, but especially Cichorium intyrbus (L.) var. foliosum and Cichorium endivia possess the significant advantage that as a result of the introduction of CMS no other properties that are characteristic of the vegetable plant are lost.

General steps of the method for obtaining CMS plants, seeds or parts of vegetable plants belonging to the Compositae family according to the invention comprise:
- isolation of protoplasts (plant cells from which the cell wall has been removed) from tissues of various vegetable plants belonging to the Compositae family, hereinafter called "acceptor", and from tissues of a different species of the Compositae family, for example Helianthus species., hereinafter called "donor" (see example 1);
- optional treatment of the protoplasts, for example by means of irradiation, cytoplasting or by means of specific chemicals, which further in the process results in an efficiency increase (see example 2);
- fusion of protoplasts (in vitro fusion of cells), for example under the influence of chemical substances, or by using electricity (see example 3);
- optionally followed by carrying out (a) process(es), which result(s) in some form of selection of hybrid cells; cells resulting from fusion of (an) acceptor protoplast(s) with (a) donor protoplast(s) (see example 4);
- followed by the culturing of cells and their regeneration to whole plants (see example 5);
- selection of individuals from the collection of regenerants thus obtained by analysis of mitochondrial, chloroplast and genomic DNA, followed by phenotypic evaluation of plant properties (see example 6)
- specific crossing of the above individuals, or their progeny, with other genotypes of the same species and other species (see example 7);
- large-scale seed production.
   The invention will be explained in more detail hereafter with reference to the examples (already briefly referred to above) discussed below, which constitute all preferred embodiments of the method according to the invention. In order not to complicate these examples unnecessarily, the examples are based on Helianthus annuus (L.) being the donor. As already explained before, the invention generally relates to a donor from the Compositae family of a species other than the vegetable plant and is consequently not limited to the donor cited referred to in the examples.
   In all the examples below operations must be carried out aseptically and all solutions, mediums (whose compositions will be described at a later stage) and instruments to be used must be sterile. Petri dishes are furthermore sealed with a film layer, such as Fuji, Nesco or parafilm.

### Example 1:

### Culturing acceptor material; in-vitro leaf of chicory, endive and/or lettuce

Wash seeds with 70% ethanol for 10 seconds, then sterilize for 10 minutes in 1% Na-hypochlorite, then wash thoroughly with sterile water. Seed aseptically in a disposable tissue culture container with approx. 75 ml of MS-medium (approx. 8 seeds/tray). Culturing at 25°C in light (16 hours light; approx. 2000 lux / 8 hours dark). After approximately two weeks the young seedlings are put in a tray with fresh MS-medium (1 seedling/tray). One month after seeding the plants are ready to be used as starting material.

### Protoplast isolation from leaf of acceptor material; chicory, endive and lettuce

Cut in-vitro leaf material (± 25 cm²) with a scalpel in a petri dish (TC, diameter 9 cm) with approx. 10 ml of PPO. Replace the solution after cutting by approx. 10 ml. of maceration solution. Incubation: stationary, dark 25°C. approx. 16 hours. Filtration through 45 µm nylon mesh, final washing and diluting the suspension with approx. 3 volumes of washing solution. Centrifuge the protoplast suspension in 15 ml centrifuge tubes for 5 min. at 600 rpm (= 63 x g). Wash pellet twice with 15 ml of washing solution, then centrifuge for 5 min. at 600 rpm. The protoplasts (pps) are now ready for fusion or for culturing, now an inactivation step, preferably an iodine acetate treatment, may be carried out, if desired.

### Culturing donor material; sunflower hypocotyls

Etiolated hypocotyls are selected as the starting material of the donor from which the protoplasts are isolated. In comparison with leaf, which is selected as "acceptor" tissue, this plant material contains a far greater amount of mitochondria. Moreover, hypocotyls do not contain any chloroplasts (they do contain some proplastides), whereas leaf cells do. This tissue selection leads to a greater chance of obtaining the correct organelle composition of a fusion product. In addition this tissue selection enables optical selection following fusion; fusion products of acceptor and donor protoplasts are recognizable.

Sterilize sunflower seeds in 1% Na-hypochlorite for 10 min., then wash thorougly with sterile water. Seed in replica dishes with MS medium. Dark, 25°C.

### Protoplast isolation from donor material; sunflower

Cut the upper half of the hypocotyls in PPO. 15 after 5 days, cut the hypocotyl parts very fine in a 9 cm TC dish with 10 ml of PPO, renew the PPO solution after cutting and allow to stand for 1 hour, dark (room temperature).

Replace the PPO solution by a maceration solution. Incubation: stationary, dark, 25°C, 16 - 18 hours (possible FITC/FDA coloration; 2 drops of the saturated stock solution, in acetone, in 10 ml of maceration solution).

Filtration through 45 µm nylon mesh, final washing with washing solution. Centrifuge for 8 min. at 600 rpm. Wash pellet again with washing solution; centrifuge for 8 min. at 600 rpm. The protoplasts (pps) are now ready for fusion. Now a nucleus inactivation/elimination step, for example irradiation or cytoplasting, may be carried out, if desired.

The culturing conditions for chicory/endive or lettuce are such, however, that the sunflower protoplasts indeed form parts and calli, but that there is no shoot regeneration.

### Example 2:

### Acceptor cytoplasm inactivation; iodine acetamide treatment

Like iodine acetate, iodine acetamide is a cytoplasm-oriented biochemical inhibitor. It is used here to inactivate the mitochondria of the acceptor protoplasts, as a result of which these cells are hardly able to survive, if at all. This gives the mitochondria of the donor in a fusion product a selective advantage over those of the acceptor, whilst in addition the continued growth of fusion products is supported in preference to that of the acceptor protoplasts by using this chemical substance. The treatment of protoplasts with iodine acetamide will for example take place in the following manner:

Stock solution 60 mM, use 1-5 mM (preferably 2.5 mM) in washing solution with a cell density of 10⁴ to 10⁶ (preferably 10⁵), incubate for 5 - 30 min. (preferably 15 min.) at a temperature of 2 - 22°C, more particularly at 4°C. Then wash the treated protoplasts very thoroughly with washing solution. Introduce the protoplasts into the fusion solution after that.

### Donor nucleus inactivation; irradiation

X-ray, gamma or ultraviolet irradiation serves the purpose of fragmenting the chromosomal DNA of the donor protoplasts, as a result of which these cells are no longer able to divide. Moreover, as a result of this the nucleus of the donor in a fusion product will have a selective disadvantage in comparison with the acceptor. The advantage of irradiation is that the protoplast membranes are not damaged too much, which is important because the fusion may have a disastrous effect on this. The disadvantage is, however, that the nucleus will remain present, be it in fragmented condition, with the attendant risk of an at least partial transmission of chromosomal DNA from the donor to the acceptor taking place.

X-ray irradiation will for example take place as follows: place pps in suspended condition in pps culture medium at a density of approx. 2.5 x 10⁶ pps/ml in a 6 cm TC petri dish (2 ml) under a X-ray source (for example Baltobloc CE 100) at a suitable distance, such that the dose will be 10 Krad per minute, for 3 - 20 minutes (preferably 5 minutes). Then wash the cells twice with washing solution.

Gamma irradiation usually takes place by means of a Cobalt 60 source. Also here the protoplasts are introduced into pps culture medium. A suitable dose is 2 - 30 Krad. Followed by washing the cells twice with washing solution.

### Donor nucleus elimination; cytoplasting

When cytoplasting takes place the nucleus is removed from the protoplast, so that the only thing that remains is a cytoplast which contains mitochondria and proplastides. There are several possibilities to effect that. For example, the difference in specific mass between the nucleus and the cytoplast may be utilized. The nucleus is literally flung out of the cell by means of high-speed centrifugation and a discontionuous (percolvsucrose) gradient, possibly in the presence of cytochalacine-B. A different, milder method is to effect strong plasmolysis during the enzym incubation, by using a maceration solution having a relatively high osmolarity (for example 0.7 M Mannitol), in that situation cytoplasts are formed spontaneously, up to as much as 40% of the cells. These cytoplasts may then be separated from the protoplasts by utilizing the difference in specific mass between protoplasts and cytoplasts. The pps/cps mixture is loaded on a percoll/mannitol gradient and refractioned by low-speed centrifugation (160 xg; 10 minutes). The success of these operations depends to a large extent on the selection of the starting material. A combination of the above-described methods is possible, if necessary. Of course the cytoplasts are washed (washing solution) after purification, and introduced into the fusion solution. A major advantage of cytoplasts is that the nucleus is eliminated, which rules out the possibility of transmission of chromosomal DNA from the donor to the acceptor. A drawback for PEG fusion is that cytoplasts have a low specific mass, as a result of which they remain afloat.

### Example 3:

### PEG fusion of chicory/endive/lettuce mesophyl protoplasts with sunflower hypotocyl protoplasts

Allow drops of 100 µl (1 - 2 for chicory, 4 - 8 for endive and lettuce) of a pps suspension having a density of 10⁶, whereby the fusion partners are mixed 1 : 1, to settle undisturbed in a fusion solution in uncoated petri dishes (9 cm diameter for chicory, 6 cm for endive and lettuce) for 15 minutes. For every drop of pps solution two droplets (approx. 20 µl) of PEG1 are charged; after 2-10 min., dependent on how fragile the pps are (mostly 4 min.), the dish is held in a sloping position and the liquid can be sucked out at the edge by means of a pasteur pipette, whilst the protoplasts remain stuck to the bottom on which they have settled. A few drops of PEG2 are carefully charged and after 3-15 min. (mostly 7 min.) they are removed in the same manner as the PEG1 solution. Do the same for PEG3, add and remove after 5 - 30 min. (mostly 10 min.). Then replenish with protoplast culture medium (10 ml of WA medium for chicory, 2 ml of AA medium for endive or 2 ml of SA medium for lettuce), and culture as described before. PEG fusion is a very suitable method for fusing large amounts of protoplasts in one go.

### Electrofusion of chicory/endive/lettuce mesophyl protoplasts with sunflower hypotocyl protoplasts

Electrofusion is to be preferred over PEG fusion when the protoplasts of both fusion parents have rather different specific masses, for example. This is for example the case when cytoplasts (see the above example 2) are used as the donor material; as a result of the absence of a nucleus the cells are relatively light-weight, which makes them remain afloat, this renders PEG fusion impossible. An advantage is that the electrofusion process makes it possible to work with great accuracy and in a very controlled manner; even fusion of only two protoplasts is possible. For this an electrofusion apparatus and a fusion chamber is necessary. The procedure may be as follows:

The mixture of donor and acceptor protoplasts is suspended in an isotonic solution (0.5 M Mannitol + 0.2 mM CaCl₂), and charged to the fusion chamber.

Then an electric field of a high-frequency alternating current (oscillation 0.3 - 1.5 Mhz) is applied, which causes the protoplasts to arrange themselves in chains; duration (10 seconds -10 minutes. The pps density (10⁴ - 2 x 10⁶) and the field strength (1 - 150 V RMS/cm) determine the length of the chains and must constantly be optimized. Then the protoplasts are exposed to (a) short, strong current surge(s), which may cause membranes which are in contact with each other to fuse. The amplitude of the pulse(s) [40 - 3000 V/cm) as well as the duration [10 microseconds - 1 millisecond], the shape [block], the number [1 - 10] and the interval of the pulse(s) have an influence on the fusion frequency and the survival of the cells. The AC field will be maintained for some time, after which the cells may be introduced into pps culture medium.

### Example 4:

### Cell sorting

It is possible to separate the desired fusion products, preferably 5 - 10% of the total number of protoplasts, from the fusion mixture by purification directly after the fusion. It stands to reason that this will benefit the efficiency of the total procedure. To that end techniques which are known per se may be used, such as micro manipulation, flowsorting or other techniques. It is not necessary, however, to use cell sorting, especially not if inactivation [for example iodine acetamide, irradiation and/or cytoplasts] are utilized. Moreover, sunflower will not regenerate under the conditions described for chicory, endive or lettuce.

### Example 5:

### Culturing of chicory protoplasts

Plate in WA-medium, the density being 5 x 10³ pps/ml, 10 ml in a 9 cm TC petri dish.

Culture at 25°C, in dark.

Diluting with WB medium must take place within 10 days after isolation, in order to prevent browning and dying of the cells. Dilute 1 : 10 in 9 cm TC petri dishes (10 ml per dish). Maintain dishes at 25°C again, but now in dimmed light (16 hours in light; approx. 500 lux / 8 hours in dark).

When the calli have a reasonable diameter (from 2 mm), approx. 1 - 2 months after protoplast isolation, they must be transferred to shoot regeneration medium; WC medium, approx. 50 calli per petri dish (diameter 9 cm) with approx. 20 ml of medium. 25°C, in light (16 hours in light; approx. 1000 lux / 8 hours in dark).

After about a month shoots may be cut from the calli and be transferred to disposable tissue culture containers (6 - 8 per tray) with approx. 75 ml of D medium for root development, in light (16 hours in light; approx. 1000 lux /8 hours in dark) at 25°C.

### Culturing of endive protoplasts

Plate in AA medium, density 10⁴ - 10⁵ pps/ml, 2 ml in a 6 cm TC petri dish.

Culture at 25°C, in dark.

When there is a division and at least part of the protoplasts have reached a 4-cell stage, diluting with WB medium, for example 1 : 2, must take place. Maintain dishes at 25°C again, but now in dimmed light (16 hours in light; approx. 500 lux / 8 hours in dark).

When the calli have a reasonable diameter (from 2 mm), approx. 2 - 4 months after protoplast isolation, they must be transferred to shoot regeneration medium; AC, approx. 50 calli per petri dish (diameter 9 cm) with approx. 20 ml of medium. 25°C, in light (16 hours in light; approx. 1000 lux / 8 hours in dark).

After about 2 - 4 months shoots may be cut from the calli and be transferred to disposable tissue culture containers (6 - 8 per tray) with approx. 75 ml of D medium for root development, in light (16 hours in light; approx. 1000 lux / 8 hours in dark) at 25°C.

### Culturing of lettuce protoplasts

Plate in SA-medium, density 2.5 x 10⁴ pps/ml, 2 ml in a 6 cm TC petri dish.

Culture at 25°C, in dark.

One week after pps isolation the first dilution with SB medium will take place, for example 1:1. and subsequently every 5 days, in order to prevent browning and dying of the cells.

When the calli have a reasonable diameter (from 2 mm), approx. 1 month after protoplast isolation, they must be transferred to shoot regeneration medium; SC medium, approx. 50 calli per petri dish (diameter 9 cm) with approx. 20 ml of medium. 25°C, in light (16 hours in light; approx. 1000 lux / 8 hours in dark).

After about two months shoots may be cut from the calli and be transferred to disposable tissue culture containers (6 - 8 per tray) with approx. 75 ml of D medium for root development, in light (16 hours in light; approx. 1000 lux / 8 hours in dark) at 25°C.

### Example 6:

### DNA analysis of mitochondria and chloroplasts

Total DNA is isolated from leaf material of the donor, acceptor, fusion products and progeny by means of a Dellaporte method (Plant Molecular Biology reporter, vol. 1, number 4 (1983)). Total DNA comprises genomic, mitochondrial and chloroplast DNA. Total DNA is digested into smaller fragments by means of restriction enzymes. The digested DNA is subsequently separated according to fragment size by means of electrophoresis in an agarose gel. After separation single-strand DNA fragments are transferred to a positive-charged membrame by means of vacuum blotting, the so-called "Southern blotting principle" (Journal Mol. Biol. 98, 503 - 517 (1975)). The various membranes comprising single-strand DNA products are hybridized with:
- mitochrondrial DNA sequences, codes: pEZMT22 and pEZMTA2;
- chloroplast DNA sequence, code: pEZCP64.

For labeling, hybridization and detection the "ECL direct nucleic acid labeling and detection system #RPN3001" (Amersham) is used.

Any changes in the mitochondrial DNA of regenerants that are detected can on the one hand be ascribed to the occurrence of specific rearrangements during the fusion and regeneration procedures that have been followed. Fusion product and progeny may likewise contain, at least partly so, the mitochondrial donor DNA. Figure 1 shows a specific fusion product and progeny thereof containing mitochondrial DNA, which is the result of specific recombination events between the mitochondrial DNA of the donor (Helianthus) and the acceptor (Cichorium).

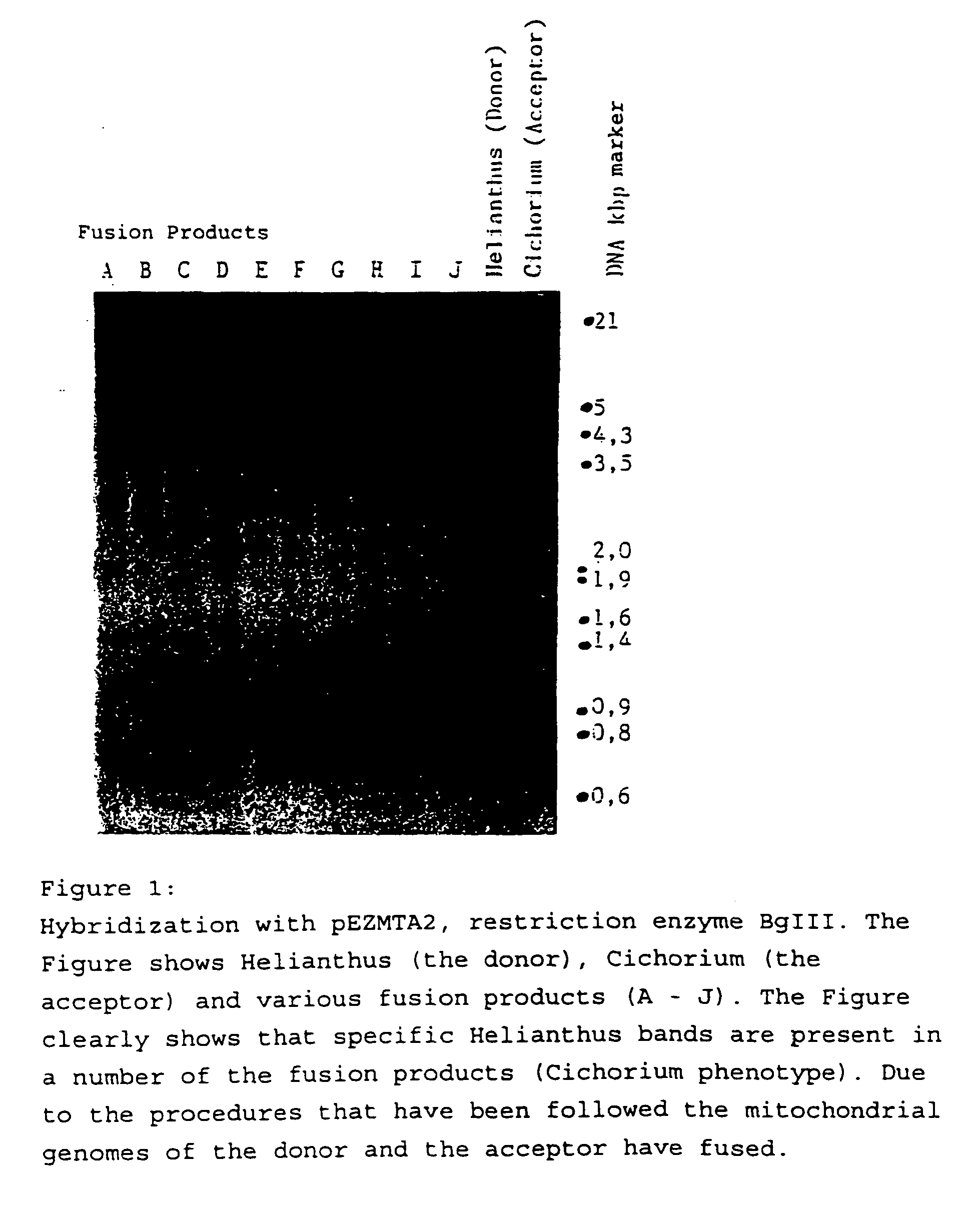

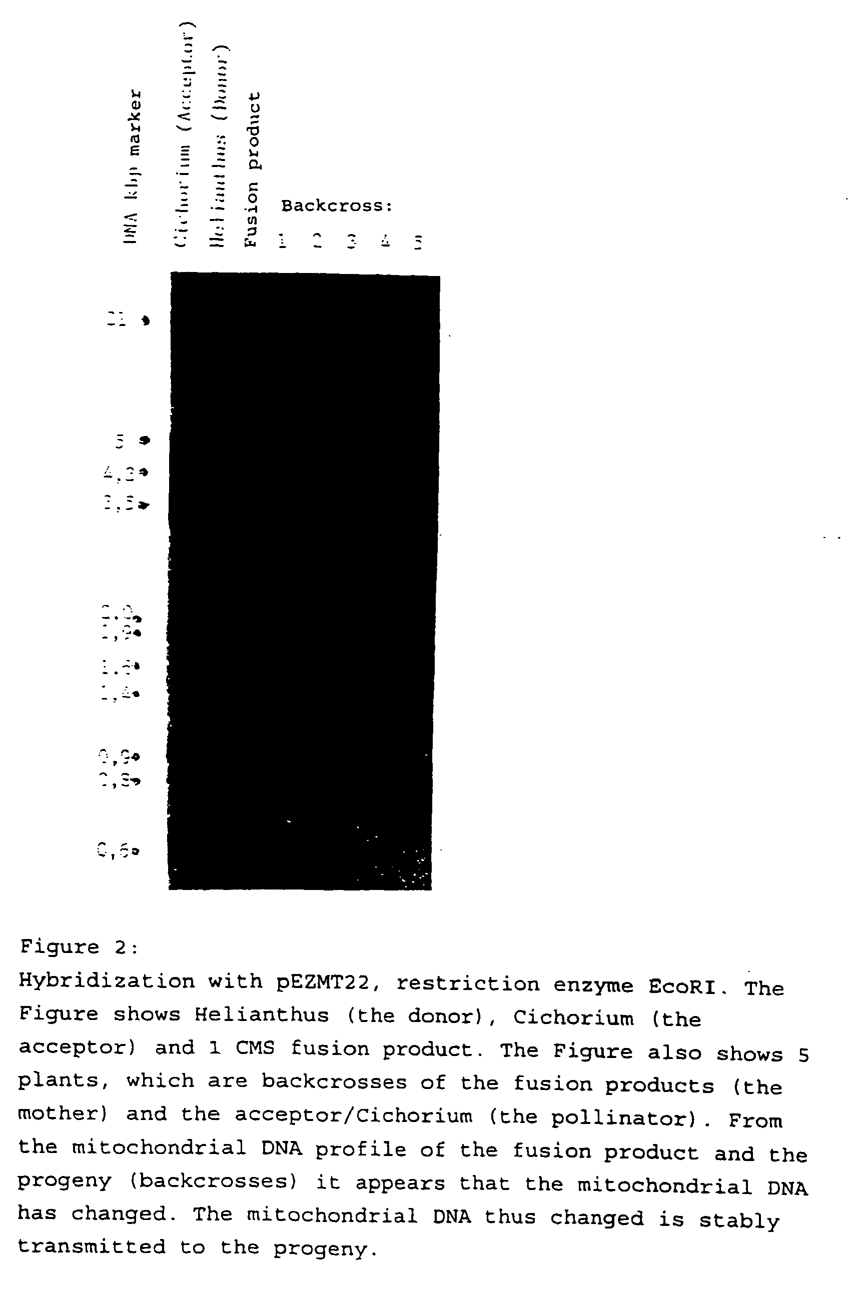

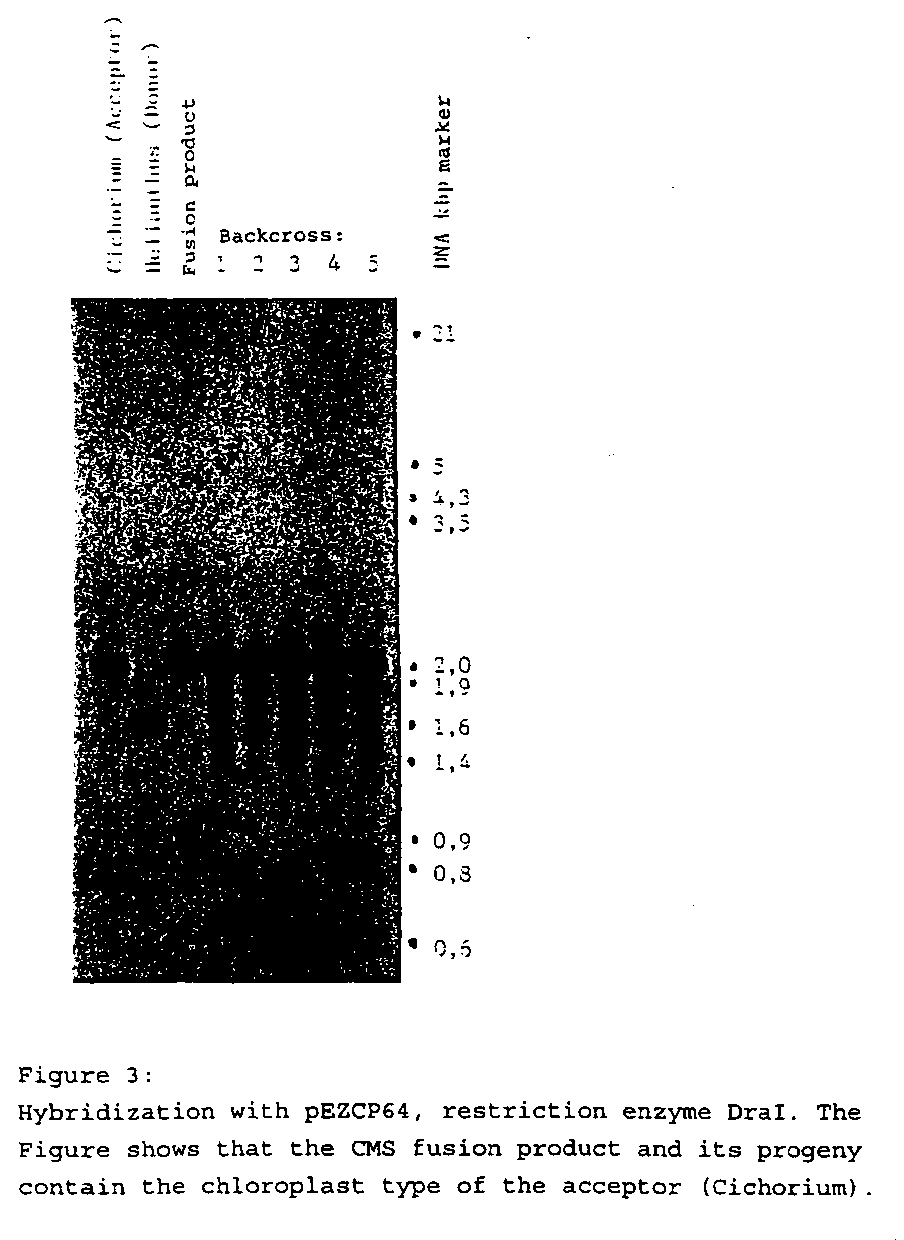

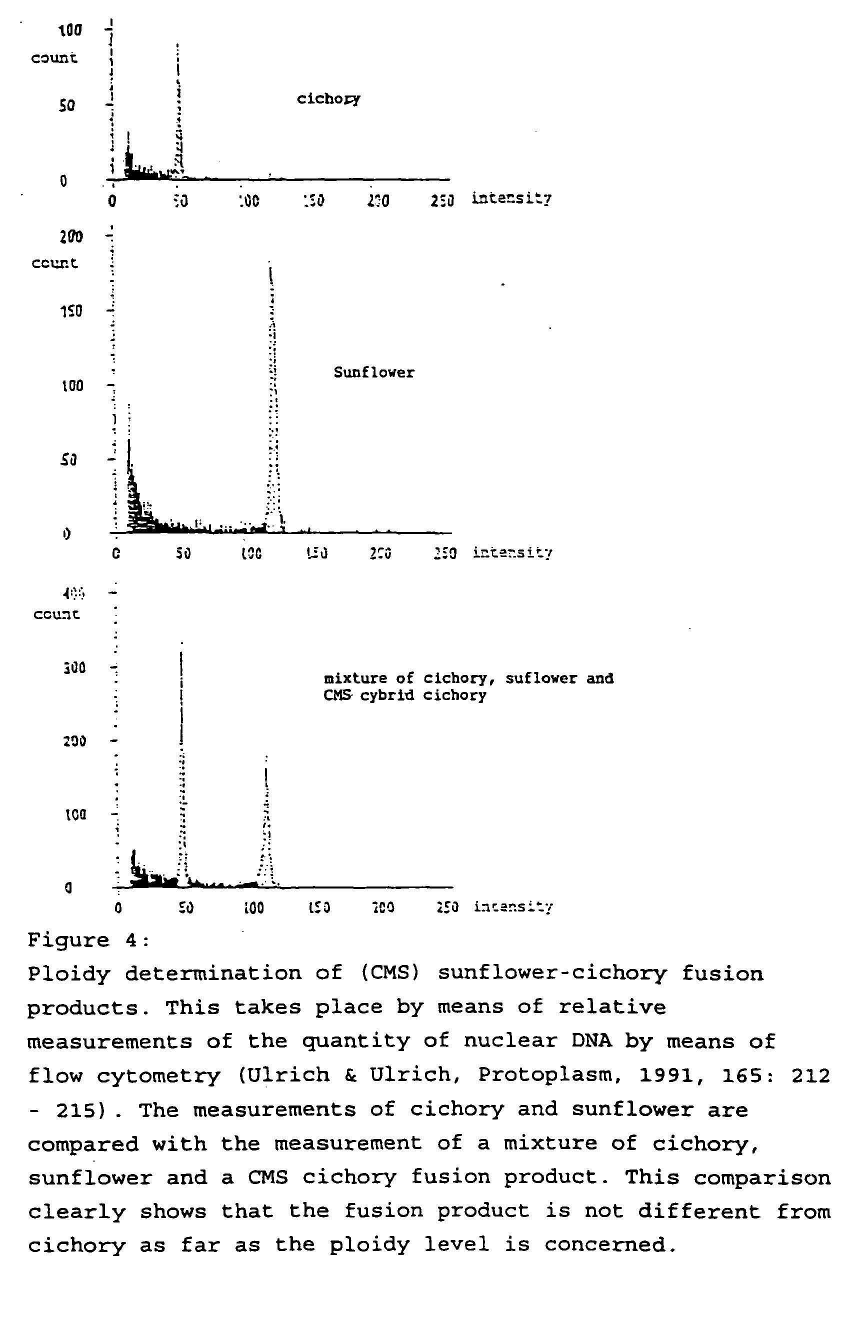

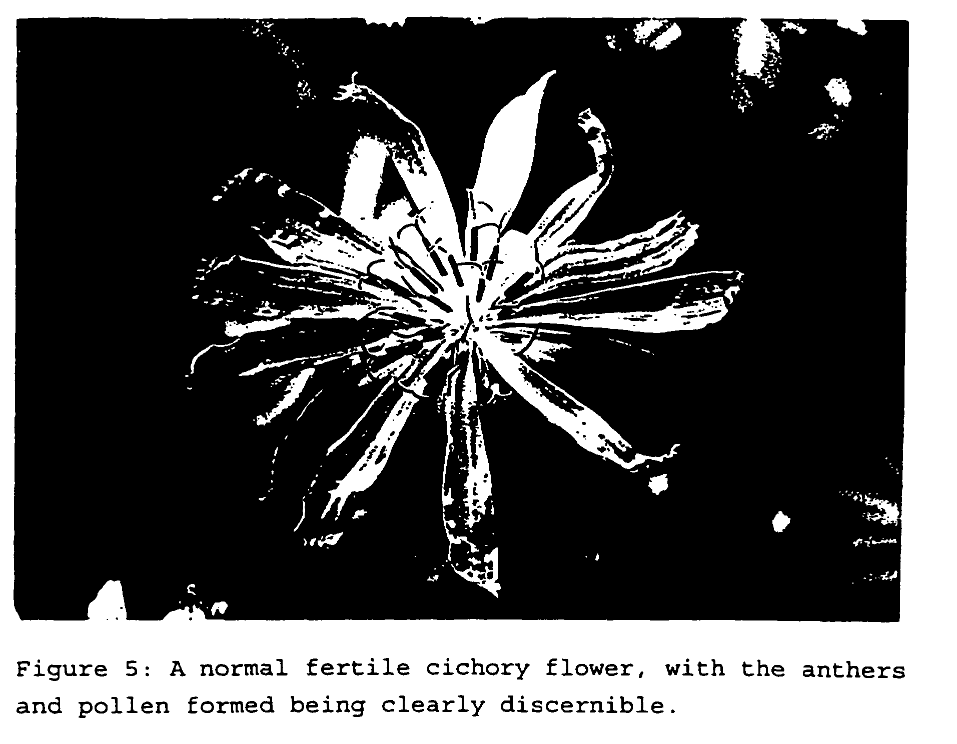

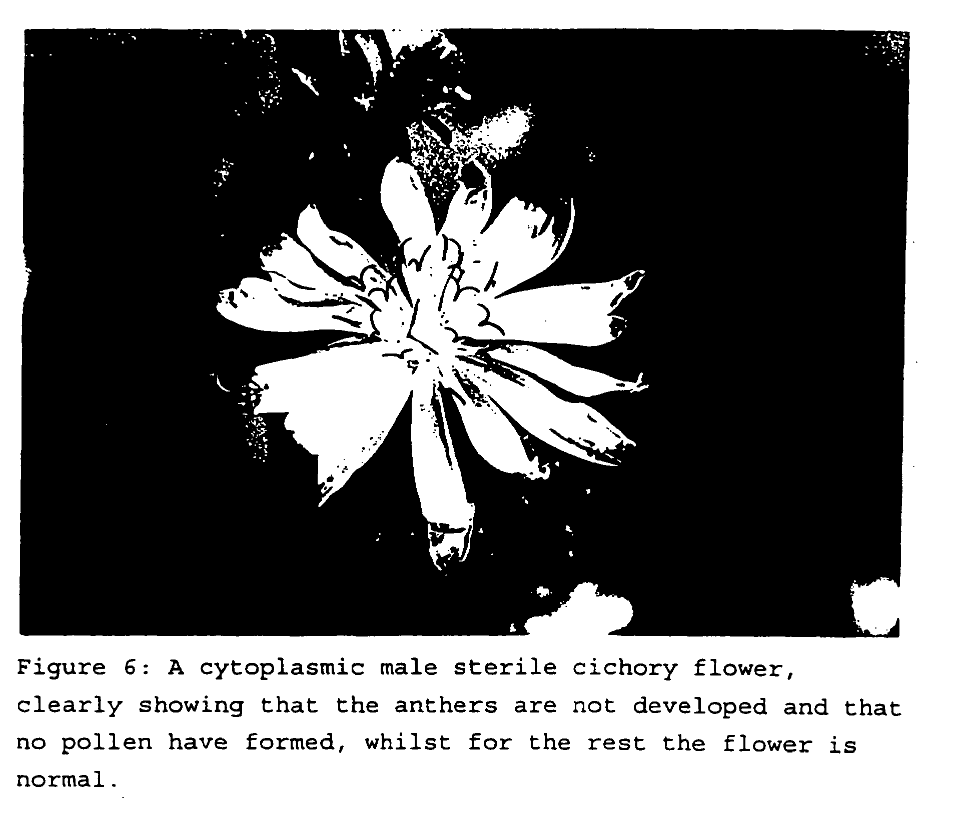

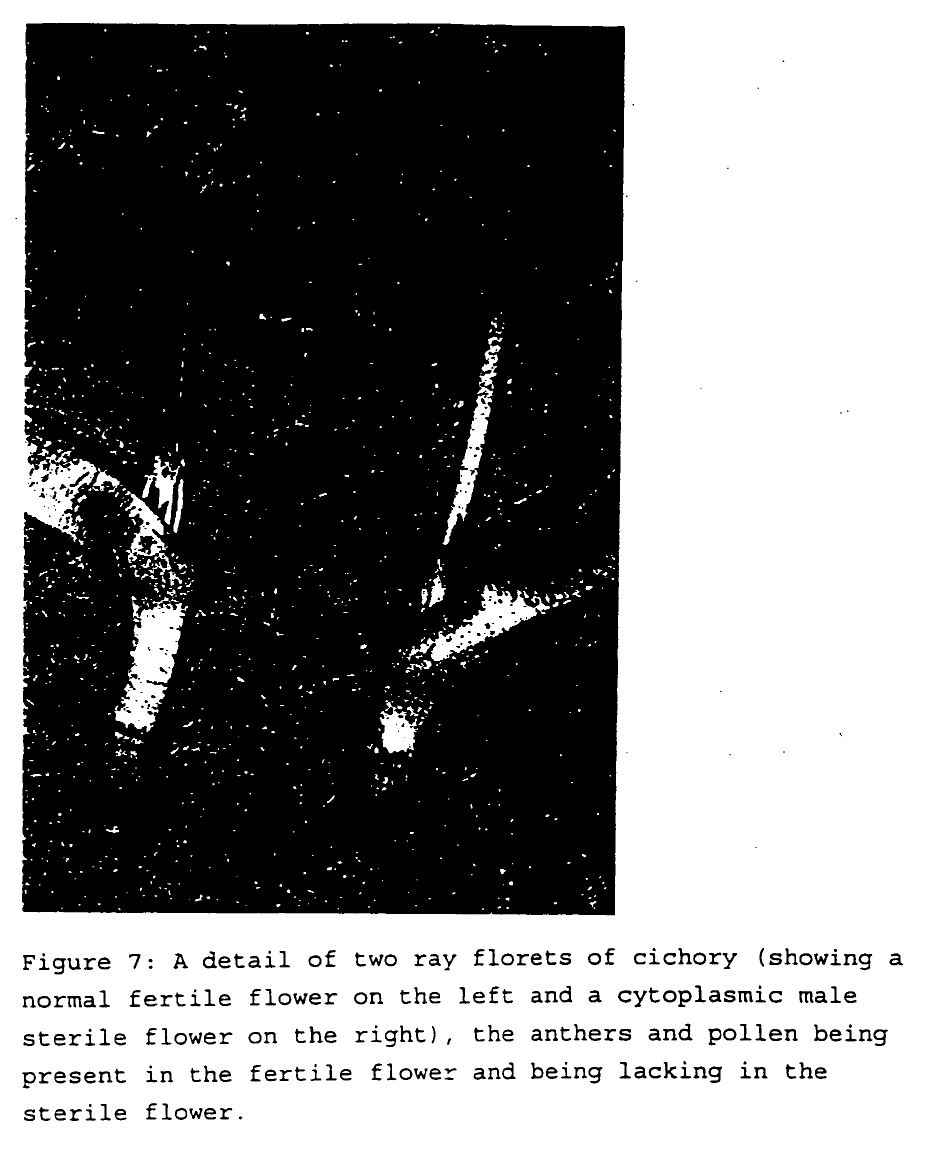

### Example 7:

### Crosses

Of course it is necessary to multiply fusion products and to adapt the genotype to the latest requirements again by means of common plant breeders' procedures. Seed has to be produced to that end. This may take place on a large scale in cages by using bees or flies, or quite specifically by manual crossing. In that case pollen of a suitable pollinator are placed on the pistils of the fusion product (or the progeny thereof), after which seeds are formed. As a result of maternal heredity all cytoplasm-coded properties, such as CMS, will be present in all progeny. In this manner it is possible to make both inter-specific and intra-specific combinaties. A good example of simple intra-specific crossing is the combination of cichory and endive; no special techniques, such as embryo rescue, are necessary to carry out this crossing.

### Media and solutions

### PPO (preplasmolysis-solution)

| | |
|---|---|
| 54,64 g/l | sorbitol |
| 7,35 g/l | CaCl₂.2H₂O |
| 0,59 g/l | MES |
| pH 5.8, | autoclave (20 minutes, 115°C) |

### Maceration solution:

### Dissolve enzymes in WA-medium

| | |
|---|---|
| Cellulase R-10 | 0,0667% |
| Driselase | 0,033% |
| Macerozyme R-10 | 0.013% |
| Cellulysine | 0,2% |
| Macerase | 0,03% |
| MES | 0,2 g/l |
| 2,4D | 0,33 mg/l |
| BAP | 0,16 mg/l |
| pH 5.6 | filter sterile (0,22µM) |

### Washing solution:

| | |
|---|---|
| CaCl₂.2H₂O | 0,2% |
| KCI | 2,5% |
| MES | 0.06% |
| pH | 5.8, autoclave |

### Fusion solution:

| | |
|---|---|
| Sorbitol | 0.15M |
| CaCl₂.2H₂O | 0,03M |
| KCI | 0,075M |
| Tris-HCl | 0,05M |
| pH | 7.2, autoclave |

### PEG1:

| | |
|---|---|
| PEG 1500 | 40% |
| Glucose | 0,3M |
| CaCl₂.2H₂O | 50mM |
| Filter sterilization | (0,22µM) |

### PEG2:

| | |
|---|---|
| PEG 1500 | 13,3% |
| Glucose | 0,1M |
| CaCl₂.2H₂O | 0,067M |
| Sorbitol | 0,067M |
| Filter sterilization | (0,22µM) |

### PEG3:

| | |
|---|---|
| PEG 1500 | 6,7% |
| Glucose | 0,05M |
| CaCl₂.2H₂O | 0,083M |
| Sorbitol | 0.083M |
| Filter sterilization | (0,22µM) |

### Abbreviations used:

- PEG: polyethylene glycol
- NAA: a-naphtalene acetic acid
- 2,4D: 2,4-dichlorophenoxy acetic acid
- BAP: 6-benzyl amino purine
- IAA: indole-3-acetic acid
- IBA: indole-3-butyric acid
- MES: (2(N-morpholino)ethanesulfonic acid
- FITC: fluorescein isothio cyanate
- FDA: fluorescein diacetate
- TC: tissue culture

### Composition of the mediums (mg/l)

| | MS | AA | WA | WB | WC | D | SA | SB | SC | AC |
|---|---|---|---|---|---|---|---|---|---|---|
| KNO₃ | 1900 | 1900 | - | - | 1900 | 950 | 1900 | 1900 | 1900 | 1900 |
| NH₄NO₃ | 1650 | 600 | - | - | 1650 | 825 | 80 | 80 | 80 | 80 |
| MgSO₄.7H₂O | 370 | 300 | 123 | 123 | 370 | 185 | 370 | 370 | 370 | 370 |
| KH₃PO₄ | 170 | 170 | 68 | 68 | 170 | 85 | 170 | 170 | 170 | 170 |
| CaCl₂.2H₂O | 440 | 600 | 440 | 440 | 440 | 220 | 440 | 440 | 440 | 440 |
| KCI | - | 300 | 750 | 750 | - | - | - | - | - | - |
| Kl | 0,83 | 0,75 | 0,75 | 0.75 | 0,83 | 0,42 | 0,83 | 0,83 | 0,83 | 0,83 |
| MnSO₄.4H₂O | 22,3 | 10 | 10 | 10 | 22,3 | 11,15 | 22,3 | 22,3 | 22,3 | 22,3 |
| H₃BO₃ | 6,2 | 3 | 3 | 3 | 6,2 | 3,1 | 6,2 | 6,2 | 6,2 | 6,2 |
| ZnSO₄.2H₂O | 8,6 | 2 | 2 | 2 | 8,6 | 4,3 | 8,6 | 8,6 | 8,6 | 8,6 |
| NaMoO₄.2H₂O | 0.25 | 0,25 | 0,25 | 0.25 | 0,25 | 0,125 | 0,25 | 0,25 | 0,25 | 0,25 |
| CuSO₄.5H₂O | 0,025 | 0,025 | 0,025 | 0,025 | 0,025 | 0,013 | 0,025 | 0,025 | 0,025 | 0.025 |
| CoCl₂.6H₂O | 0,025 | 0,025 | 0,025 | 0,025 | 0,025 | 0,013 | 0,025 | 0,025 | 0,025 | 0,025 |
| Fe-EDTA | 43 | 43 | 43 | 43 | 43 | 21,5 | 43 | 43 | 43 | 43 |
| Thiamiac-HCl | 0,1 | 10 | 10 | 10 | 0.1 | 0,05 | 0,5 | 0.5 | 0,5 | 0,5 |
| pyridoxine-HCl | 0,5 | | 1 1 1 | | 0,5 | 0.25 | 0,5 | 0,5 | 0,5 | 0,5 |
| Nicotine acid | 0,5 | 1 | 1 | 1 | 0,5 | 0,25 | 0,5 | 0,5 | 0,5 | 0,5 |
| Ascorbic acid | - | 2 | 2 | 2 | - | - | - | - | - | - |
| Sodium pyruvate | - | 20 | 20 | 20 | - | - | - | - | - | - |
| Citric acid | - | 40 | 40 | 40 | - | - | - | - | - | - |
| Malic acid | - | 40 | 40 | 40 | - | - | - | - | - | - |
| Fumaric acid | - | 40 | 40 | 40 | - | - | - | - | - | |
| Glycine | 2 | - | - | - | 2 | 1 | 2 | 2 | 2 | 2 |
| Fructose | - | 250 | - | - | - | - | - | - | - | - |
| Ribose | - | 250 | - | - | - | - | - | - | - | - |
| Xylose | - | 250 | - | - | - | - | - | - | - | - |
| Mannose | - | 250 | - | - | - | - | - | - | - | - |
| Rhamnose | - | 250 | - | - | - | - | - | - | - | - |
| Cellobiose | - | 250 | - | - | - | - | - | - | - | - |
| Sorbitol | - | 250 | - | - | - | - | - | - | - | - |
| Mannitol | - | 250 | 80000 | 80000 | - | - | 91000 | - | - | - |
| Inositol | 100 | 100 | 100 | 100 | 100 | 50 | 100 | 100 | 100 | 100 |
| Sucrose | 10000 | 250 | 20000 | 20000 | 10000 | 10000 | 10000 | 10000 | 20000 | 10000 |
| Glucose | - | 68400 | - | - | - | - | - | - | - | - |
| Casamino acid | - | 250 | - | - | - | - | - | - | - | - |
| Coconut water ml/l | - | 20 | - | - | - | - | - | - | - | - |
| AGAR | 8000 | - | - | - | 8000 | 8000 | - | - | 8000 | 8000 |
| TWEEN-20 | - | - | 8 | - | - | - | - | - | - | - |
| Glutamine | - | - | 750 | 750 | - | - | - | - | - | - |
| Soluble starch | - | - | - | - | - | 10000 | - | - | - | - |
| NAA | - | 0,1 | - | 0,1 | - | - | 0,1 | 0,5 | 0,5 | - |
| 2,4-D | - | 0,25 | - | - | - | - | - | - | - | - |
| IAA | - | - | 1,0 | - | - | - | - | - | - | - |
| Zeatine | - | - | 1,0 | - | 1.0 | - | - | - | - | - |
| BAP | - | 0,1 | - | 1,0 | 0,5 | - | 0,5 | 0,5 | 1,0 | 0,1 |
| IDA | - | - | - | - | - | - | - | - | - | 0,01 |
| pH | 5,7 | 5,6 | 5,8 | 5,8 | 5,7 | 5,7 | 5,8 | 5,8 | 5,8 | 5,8 |

| | | | | | | | | | | |
|---|---|---|---|---|---|---|---|---|---|---|
| N.B. Autoclave medium with AGAR; 20 minutes. 115°C Filter sterilization medium without AGAR: 0,22µM | | | | | | | | | | |

## Claims

1. A vegetable plant belonging to the Compositae family, the cytoplasm of which is provided with mitochondria comprising DNA which at least partially originates from a different species of the Compositae family, and which is the carrier of stably expressable cytoplasmic male sterility (CMS), whose cells comprise species-specific chloroplast and nuclear genome which are normal for the vegetable plant.

2. A plant according to claim 1, wherein said plant is phenotypically cytoplasmic male sterility.

3. A plant according to claim 1 or 2, wherein the other species of the Compositae family has been selected from the group consisting of Helianthus.spp, and Cirsium spp.

4. A plant according to claim 3, wherein the other species of the Compositae family Helianthus annuus (L.) with CMS of the petiolaris type.

5. A plant according to claim 1 or 2, wherein the other species of the Compositae family has been selected from the group consisting of Chrysanthemum Senecio Centaurea, Sonchus, Hiëracium, Tagetes, Dahlia and Aster.

6. A plant according to any one of the preceding claims 1-5, wherein the vegetable plant has been selected from the group consisting of Cichorium intybus (L.), var. foliosum (Hegi), Cichorium endivia (L.), Lactuca sativa (L.), Scorzonera hispanica L.), Cynara scolymus (L), and Taraxacum officinale (L.).

7. Process for producing a vegetable plant belonging to the Compositae family comprising the step of providing the cytoplasm thereof with mitochondria having DNA which at least partially originates from a different species of the Compositae family, and which is the carrier of stably expressable cytoplasmic male sterility (CMS), as well as providing the cytoplasm thereof with species-specific chloroplast and nuclear genome, wherein
- the vegetable plant has been selected from the group consisting of Cichorium intybus (L.) var. foliosum (Hegi), Cichorium endivia (L.), Lactuca sativa (L.), Scorzonera hispanica (L.), Cynara scolymus (L), and Taraxacum officinale (L.); and
- the other species of the Compositae family has been selected from the group consisting of Helianthus spp., Cirsium spp., Chrysanthemum, Senecio Centaurea, Sonchus. Hiëracium, Tagetes, Dahlia and Aster; and
- use is made of irradiation for inactivating the donor nucleus.

## Patentansprüche

1. Pflanze, die zur Familie der Compositae gehört, deren Zytoplasma mit Mitochondrien versehen ist, welche DNA umfassen, die wenigstens teilweise von einer unterschiedlichen Spezies der Familie der Compositae stammt und die der Träger von stabil exprimierbarer zytoplasmatischer Pollensterilität (CNS) ist, wobei deren Zellen Spezies-spezifisches Chloroplasten- und Kerngenom umfassen, die für die Pflanze normal sind.

2. Pflanze gemäß Anspruch 1, wobei der Phänotyp besagter Pflanze zytoplasmatische Pollensterilität ist.

3. Pflanze gemäß Anspruch 1 oder 2, wobei die andere Spezies der Familie der Compositae ausgewählt ist aus der Gruppe, bestehend aus Helianthus spp. und Cirsium spp.

4. Planze gemäß Anspruch 3, wobei die andere Spezies der Familie der Compositae Helianthus annuus (L.) mit CMS vom Petiolaris-Typ ist.

5. Pflanze gemäß Anspruch 1 oder 2, wobei die andere Spezies der Familie der Compositae ausgewählt ist aus der Gruppe, bestehend aus Chrysanthemum, Senecio, Centaurea, Sonchus, Hieracium, Tagetes, Dahlia und Aster.

6. Pflanze gemäß einem der vorangehenden Ansprüche 1 bis 5, wobei die Pflanze ausgewählt ist aus der Gruppe, bestehend aus Cichorium intybus (L.), var. foliosum (Hegi), Cichorium endivia (L.), Lactuca sativa (L.), Scorzonera hispanica (L.), Cynara scolymus (L.) und Taraxacum officinale (L.).

7. Verfahren zur Herstellung einer Pflanze, die zur Familie der Compositae gehört, umfassend den Schritt des Bereitstellens des Zytoplasmas davon mit Mitochondrien, welche DNA umfassen, die wenigstens teilweise von einer unterschiedlichen Spezies der Familie der Compositae stammt und die der Träger von stabil exprimierbarer zytoplasmatischer Pollensterilität (CMS) ist, sowie das Bereitstellen des Zytoplasmas davon mit Speziesspezifischem Chloroplasten- und Kerngenom, wobei
- die Pflanze ausgewählt wird aus der Gruppe, bestehend aus Cichorium intybus (L.), var. foliosum (Hegi), Cichorium endivia (L.), Lactuca sativa (L.), Scorzonera hispanica (L.), Cynara scolymus (L.) und Taraxacum officinale (L.); und
- die andere Spezies der Familie der Compositae ausgewählt wird aus der Gruppe, bestehend aus Helianthus spp., Cirsium spp., Chrysanthemum, Senecio, Centaurea, Sonchus, Hieracium, Tagetes, Dahlia und Aster; und
- von Bestrahlung zur Inaktivierung des Donor-Nukleus Gebrauch gemacht wird.

## Revendications

1. Végétal appartenant à la famille Compositae, dont le cytoplasme est pourvu de mitochondries comprenant de l'ADN qui a au moins partiellement pour origine une autre espèce de la famille Compositae, et qui est le porteur de la stérilité cytoplasmique mâle (CMS) pouvant être exprimée de façon stable, dont les cellules comprennent un génome nucléaire et un génome chloroplastidial spécifiques de l'espèce qui sont normaux pour le végétal en question.

2. Végétal selon la revendication 1, induisant phénotypiquement la stérilité cytoplasmique mâle dans le végétal.

3. Végétal selon la revendication 1 ou 2, dans lequel l'autre espèce de la famille Compositae a été choisie parmi le groupe comprenant Helianthus spp. et Cirsium spp.

4. Végétal selon la revendication 3, dans lequel l'autre espèce de la famille Compositae est Helianthus annuus (L.) avec CMS du type petiolaris.

5. Végétal selon la revendication 1 ou 2, dans lequel l'autre espèce de la famille Compositae a été choisie parmi le groupe comprenant Chrysanthemum, Senecio, Centaurea, Sonchus, Hiëracium, Tagetes, Dahlia et Aster.

6. Végétal selon l'une quelconque des revendications précédentes 1 à 5, obtenu à partir d'un végétal choisi parmi le groupe comprenant Cichorium intybus (L). var. foliosum (Hegi), Cichorium endivia (L.), Lactuca sativa (L.), Scorzonera hispanica (L.), Cynara scolymus (L.) et Taraxacum officinale (L.).

7. Procédé de production d'un végétal appartenant à la famille Compositae, comprenant l'étape consistant à pourvoir son cytoplasme d'une mitochondrie comprenant de l'ADN qui a au moins partiellement pour origine une espèce différente de la famille Compositae, et qui est le porteur de la stérilité cytoplasmique mâle (CMS), pouvant être exprimée de façon stable, et à pourvoir son cytoplasme d'un génome nucléaire et chloroplastidial spécifique de l'espèce, tel que
- le végétal a été sélectionné dans le groupe constitué par Cichorium intybus (L.) var. foliosum (Hegi), Cichorium endiva (L.), Lactuca sativa (L.), Scorzonera hispanica (L.), Cynara scolymus (L.) et Taraxacum officinale (L.), et
- l'autre espèce de la famille Compositae a été sélectionnée dans le groupe constitué par comprenant Helianthus spp., Cirsium spp., Chrysanthemum, Senecio, Centaurea, Sonchus, Hiëacium, Tagetes, Dahlia et Aster, et
- on utilise une irradiation pour inactiver le noyau du donneur.
